# EUROPEAN PATENT APPLICATION

(11) **EP 1 125 946 A1**
(43) Date of publication of application: **22.08.2001**
(21) Application number: 00200567.6
(22) Date of filing: 18.02.2000
(51) Int. Cl.: C07K 14/47, C12N 9/16, A61K 38/18, A61K 38/46, C12Q 1/34, A61P 43/00

(54) **Cell adhesion therapy through modulation of a Rap family member or of a GEF (guanine nucleotide exchange factor)**

(71) Applicant: Universitair Medisch Centrum Utrecht, 3584 CX Utrecht (NL)
(72) Inventor: Bos, J.L., 3584 CX Utrecht (NL); de Rooy, J., 3584 CX Utrecht (NL)
(74) Representative: Ottevangers, Sietse Ulbe

(57) **Abstract**

The invention relates to the field molecular biology, more specifically to the field of signal transduction in the control of cell adhesion. The invention provides a method for modulating cell adhesion comprising modulating a protein belonging to the Rap family of small GTPases or comprising modulating a guanine nucleotide exchange factor for a protein belonging to the Rap family of small GTPases.

## Description

The invention relates to the field of molecular biology, more specifically to the field of signal transduction in the control of cell adhesion.

Modulation of cell adhesion is a major challenge in medicine. e.g. anti-adhesion therapy may be highly beneficial to prevent among others infiltration of leukocytes in acute inflammations, rheumatoid arthritis, ischaemic reperfusion injury, and auto-immune diseases. Modulation of adhesiveness of tumour cells may inhibit invasiveness and metastatic spread and modulation of adhesiveness of endothelial cells may affect angiogenesis. Compounds central to adhesion are integrins, and the role of integrins in a variety of disease processes where anti-adhesion therapy may be useful have been reviewed extensively. (Ruoslathi, 1999, Hughes and Pfaff, 1998, Kolanus and Seed, 1997 and references therein).

Integrins are a family of transmembrane, heterodimeric cell adhesion molecules consisting of an alpha and a beta chain that function as receptor for extracellular matrix components. There are a large number of different alpha and beta chains, and the combination determines the specificity of the receptor. Integrins can modulate their adhesive capacity by a complex mechanism including comformational changes to increase affinity and clustering to increase avidity. The mechanism by which the cell regulates integrin function (inside-out signalling) is largely unclear (see below). In addition, when integrins interact with the extracellular matrix, they transduce a signal into the cell (outside-in signalling). A large variety of signalling events occurs after ligand binding, including the activation of FAK, Src, Ras etc.

To modulate or regulate integrin-mediated adhesion a cell has at least four different ways to control integrin functioning. Firstly, expression of the integrin. Secondly the translocation of integrins from intracellular vesicle to the plasma membrane, Thirdly, the activation of integrins to increase the affinity for the ligand and fourthly the clustering of integrin to increase binding capacity (avidity). Depending on the integrin and the cell types one of more of these mechanisms may predominate. Generally, integrin activation is induced by extracellular stimuli that activate other receptors on the cell surface. For instance, thrombin rapidly activates integrin aIIbb3 on human platelets (Franke et al., 1997). A large number of studies have been performed trying to elucidate the mechanism of "inside-out" signalling. These studies resulted in the peculiar finding that, although in a variety of primary cells, such as neutrophils and lymphocytes, this process clearly occurs, in most cell lines this regulation is lost. This may be due to the fact that most cell lines are tumor cell lines. Pharmacological studies have revealed that both phosphatidyl inositol-3-kinase (PI3K) and protein kinase C (PKC) are involved in the regulation of most integrins. Furthermore a large variety of proteins that interact with specific alpha or beta-chains have been identified which may be involved in the activation of integrins (Kolanus and Seed, 1997).

Recently came the finding that small GTPases may be involved in inside-out signalling. The first clear example was the work of Ruoslathi and coworkers (Zhang et al., 1996), showing introduction of an active form of R-ras results in increased adhesion of aIIbb3. Furthermore, they show that inhibition of R-ras using a dominant negative R-ras mutant results in a decreased adhesion. Furthermore, it was shown that R-ras was regulated in a for GTPases very peculiar manner, i.e. activation of the Eph receptor induces tyrosine phosphorylation of the effector domain of R-ras, resulting in inactivation.

Also Ras has been implicated in the control in inside-out regulation of integrins (Shibayama et al., 1999). In T-cells dominant negative Ras inhibits TCR-induced adhesion to ICAM, suggesting that Ras plays a positive role in integrin signalling. However, in CHO cells Ras inhibits activation of integrins and the model is proposed that R-ras and Ras operate in distinct pathways, one positive (R-ras) and one negative pathway (Ras) (Hughes et al, 1997). However, little to none is known about how these small GTPases regulate integrin activation (Hughes and Pfaff, 1997).

The invention provides a method for modulating or regulating cell adhesion comprising modulating a protein belonging to the Rap family of small GTPases, and thus provides targets for drugs that modulate adhesion of cells. These targets are intermediates of the Rap signal transduction pathway, among others guanine nucleotide exchange factors for Rap1. Compounds affecting these targets are beneficial for a large variety of diseases, among others, ischaemic reperfusion injury, auto-immune disease, acute inflammation, rheumatoid arthritis and cancer. The invention provides among others access to and insight in the role of the Rap signalling pathway in the control of integrin-mediated events. These integrin-mediated events control cell adhesion, cell migration, cell proliferation and cell survival.

The invention also provides the inhibition of the Rap signalling pathway by various means which results in the inhibition of integrin activation. The inhibitors provides by this invention are compounds or drugs that inhibit either Rap or guanine nucleotide exchange factors for Rap.

The invention also provides a method for modulating cell adhesion comprising modulating a guanine nucleotide exchange factor for a protein belonging to the Rap family of small GTPases, and thus also provides among others access to and insight in the mechanism by which guanine nucleotide exchange factors for Rap are regulated. From our work we have established that a number of these guanine nucleotide exchange factors are regulated by auto-inhibitory domains that needs to be released from inhibition by small molecules. We established the mechanism of activation for Epac, Epac2, members of CalDAG-GEF family and the PDZ-GEF family of guanine nucleotide exchange factors. Our invention provides that these guanine nucleotide exchange factors are ideal targets for drugs modulating cell adhesion.

The invention provides a method according to the invention comprising modulating said cell adhesion by modulating integrin-mediated events in said cell adhesion, in that Rap and guanine nucleotide exchange factors for Rap are targets for compounds to modulate cell adhesion through among others integrins, in particular by modulating the auto-inhibitory domain of a guanine nucleotide exchange factor for a protein belonging to the Rap family of small GTPases. Of course, the invention also provides use of a modulator of a protein belonging to the Rap family of small GTPases for the preparation of a medicament for adhesion or anti-adhesion therapy or use of a modulator of a guanine nucleotide exchange factor for a protein belonging to the Rap family of small GTPases for the preparation of a medicament for adhesion or anti-adhesion therapy, in particular wherein said adhesion or anti-adhesion therapy comprises modulating integrin-mediated events, for example wherein said modulator comprises a modulator of the auto-inhibitory domain of a guanine nucleotide exchange factor of a protein belonging to the Rap family of small GTPases.

Particularly the guanine nucleotide exchange factors for Rap are suitable targets since they are accessible to small molecules. Rap stands for a family of proteins, which consists of the proteins Rap1a, Rap1b, Rap2a and Rap2b. A guanine nucleotide exchange factor for Rap is any protein that elevates the release of GDP from Rap1 by direct physical interaction between the guanine nucleotide exchange factor and Rap. These guanine nucleotide exchange proteins include at least C3G, three members of the Epac family, three members of the CalDAG-GEF family and two members of the PDZ-GEF family

A GTPase activating protein for Rap is any protein that increases the hydrolysis rate of GTP when bound to Rap. These GTPases activating proteins include at least RapGAP and RapGAPII and Spa1. An effector of Rap is any protein that binds directly to Rap and functions in mediating signals from Rap to a biological response. A modulator of Rap is any proteins, lipid or natural compound that influences the functioning of the Rap signalling pathway

The Rap signalling pathway comprises proteins, lipids and natural compounds that constitute a signalling pathway involving Rap.

The invention also provides a method for identifying a modulator of a protein belonging to the Rap family of small GTPases or of modulator of a guanine nucleotide exchange factor for a protein belonging to the Rap family of small GTPases comprising providing for binding of a nucleotide or nucleotide analogue to a protein belonging to the Rap family of small GTPases, incubating said protein in the presence of a guanine nucleotide exchange factor of a protein belonging to the Rap family of small GTPases and incubating said protein in the presence of a candidate modulator compound and measuring release of said nucleotide or nucleotide analogue from said protein belonging to the Rap family of small GTPases. For example, the invention provides a method for detecting a modulator that activates (activator) using a method wherein activation of said release is measured, e.g. by loading Rap1 with mantGDP, incubating it in the presence of guanine nucleotide exchange factors containing the auto-inhibitory domain, adding test compounds (candidate modulator compounds)and measuring the release of mantGDP spectroscopically. In another example, the invention provides a method for detecting a modulator that inhibits (inhibitor) using a method wherein activation of said release is measured, e.g. by loading Rap1 with mantGDP, incubating it in the presence of guanine nucleotide exchange factors containing the auto-inhibitory domain and in the presence of an (natural) activator, adding test compounds (candidate modulator compounds)and measuring the inhibition of the release of mantGDP spectroscopically.

In such a way, the invention provides a modulator obtainable by a method according to the invention. Such modulators are for example compounds, which modulate the activity of a guanine nucleotide exchange factor for Rap, Rap, a GTPase activating protein for Rap, an effector for Rap or a modulator of the Rap signalling pathways and which are useful in the modulation of cell adhesion, exemplified by compounds which modulate the functioning of the autoinhibitory domain of a guanine nucleotide exchange factor for Rap1a and Rap1b, such as short peptides, or even large molecules such as antibodies reactive with said autoinhibitory domain, which results in the modulation of integrin-mediated cell adhesion. The invention provides use of such a modulator in a method for modulating cell adhesion or use for the preparation of a medicament, in particular for for adhesion or anti-adhesion therapy, and provides such a medicament. The invention is further explained in the detailed description without limiting the invention thereto

### Detailed description

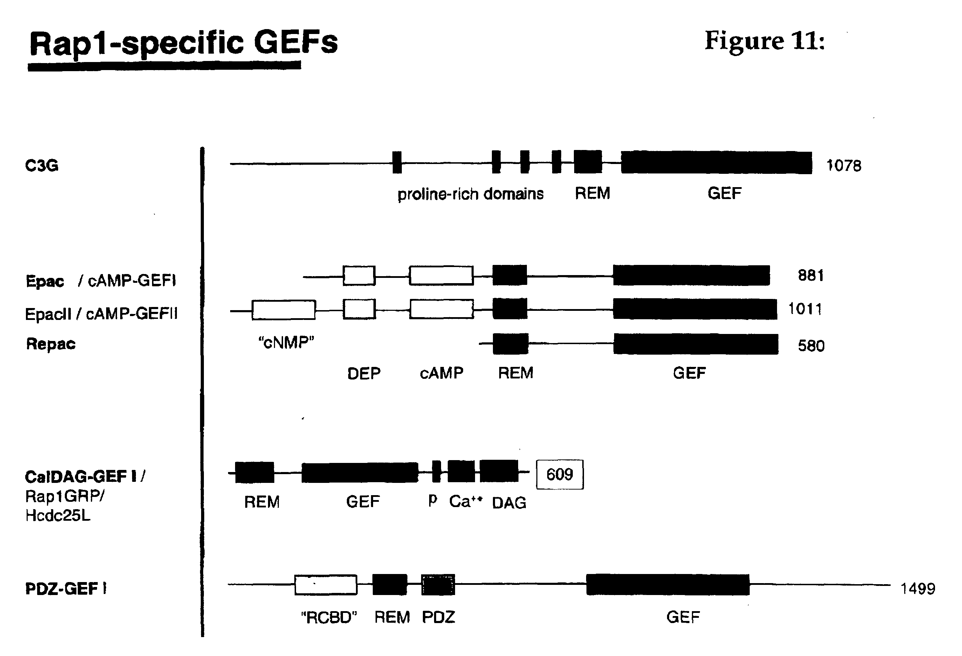

The small GTPase Rap1, a close relative of Ras, was first described as a revertant of K-ras transformed cells. Since the effector domain of Rap1 is very similar to the effector domain of Ras, the model was proposed that Rap1 interacts with the same effectors as Ras, but inhibit, rather than activate these effectors. This antagonistic effect on Ras signalling is still one of the major models for the function of Rap1 that appear in the literature. However, from our work of the last few years this model is heavily challenged. Using newly developed assays for the activation of Rap1 we observed that Rap1 is rapidly activated by a large variety of stimuli, including stimuli that also activate Ras. Common second messengers, like calcium, diacylglycerol and cAMP, activate a number of GEF for Rap1 directlyresulting in the activation of Rap1. (See Figure 11) Also the downregulation of Rap1 is regulated. For instance, the alpha-subunit of Gi directly associates with and inactivates RapGAP. Importantly, when these stimuli are used to activate Rap1, we did not observe inhibition of the Ras signalling pathway. From these and other results we concluded that Rap1 more likely has a distinct function. We therefore embarked on the identification of this distinct pathway and found that at least one of the pathways in which Rap1 is involved is the regulation of integrin-mediated adhesion.

### Example 1

### Rap1 mediates inside-out signalling to regulate integrin-mediated cell adhesion

Integrin-mediated leukocyte adhesion is a critical aspect of leukocyte function that is tightly regulated by diverse stimuli, including chemokines, antigen receptors, and adhesion receptors. How cellular signals from CD31 and other adhesion amplifiers are integrated with those from classical mitogenic stimuli to regulate leukocyte function remains poorly understood. Several reports suggest a role for Ras family GTPases in the control of integrin-mediated adhesion. Ras superfamily GTPases cycle between inactive GDP-bound forms and active GTP-bound forms, and exchange for GTP and hydrolysis of GTP to GDP are catalyzed by guanine nucleotide exchange factors (GEFs) and GTPase activating proteins (GAPs), respectively (Bos, 1997; Bos 1998). Use of active and dominant-negative mutants of H-Ras and R-Ras has revealed that these enzymes can regulate coupling of betal and beta2 integrin-dependent adhesion to T cell receptor (TCR/CD3), interleukin-3, and chemokine receptor signaling in T lymphocytes and pro-B cell leukemic cell lines (Liu et al., 1999; O' Rourke et al., 1998; Shibayama et al., 1999; Tanaka et al., 1999; Zhang et al., 1996). We have examined whether CD31 stimulates integrin-dependent adhesion via an intracellular signaling pathway, and if so, whether members of the Ras family were involved in this process. We identify the small GTPase Rap1 as a critical mediator of this effect. Importantly, CD31 selectively activated the small Ras-related GTPase Rap1, but not Ras, R-Ras or Rap2. An activated Rap1 mutant stimulated T lymphocyte adhesion to ICAM and VCAM, as did the Rap1 guanine nucleotide exchange factor C3G and a catalytically inactive mutant of RapGAP. Conversely, negative regulators of Rap1 signaling blocked CD31-dependent adhesion. These findings identify a novel important role for Rap1 in regulating ligand-induced cell adhesion and suggest that Rap1 may play a more general role in coordinating adhesion-dependent signals during leukocyte migration and extravasation.

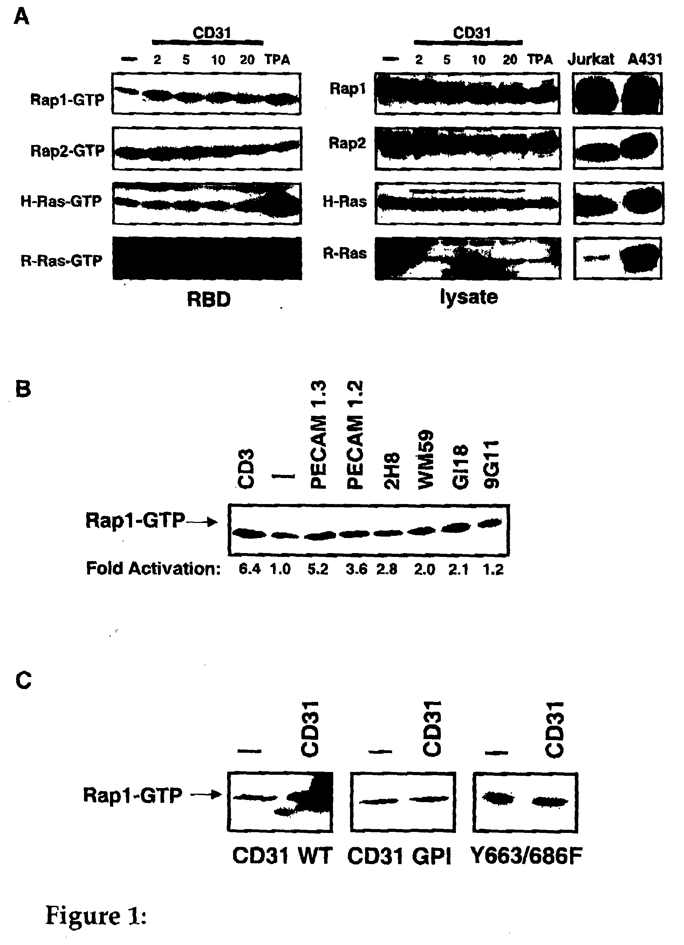

To address which Ras family members, if any, might participate in CD31 signaling, we precipitated endogenous GTPases from lysates of CD31-stimulated Jurkat with GST fusion proteins of Ras family-binding domains (RBDs) of Raf and RalGDS, which bind with high selectivity and specificty to activated Ras and Rap GTPases, respectively (de Rooij and Bos, 1997; Franke et al., 1997). Immunoblotting of precipitated GTPases provides a qualitative representation of GTPase activation status following cell stimulation, corresponding to quantitative changes detected using classical GDP/GTP-binding ratio techniques. Time-course analyses of Jurkat cells treated with anti-CD31 antibodies or TPA (Figure 1A) surprisingly revealed a rapid activation of the Ras-related GTPase Rap1 by CD31, which was maximal 2-5 minutes post-stimulation and decreased slowly toward basal levels after 20 minutes. In contrast, while both Rap1 and Ras were activated by TPA, CD31-dependent stimulation of Rap1 was highly selective and no activation of Rap2, Ras or R-Ras was noted at any point during this time-course. Both Rap1 and Ras, but not the other GTPases tested, were also activated by TCR stimulation (data not shown). GTP-bound R-Ras was detected in Raf-RBD pull-downs only following longer exposures of films, reflecting the relatively low levels of R-Ras protein expression in Jurkat. R-Ras protein expression was at least 10-fold lower in Jurkat cells as compared to A431 fibroblasts, while Rap1, Rap2, and Ras levels were relatively equivalent (right panels, last two lanes).

Functional anti-CD31 antibodies against CD31 extracellular domains 1 and 6 (PECAM 1.2, PECAM 1.3 and 2H8), activated Rap1 more potently than non-functional antibodies (WM59, 9G11, GI18) (Figure 1B), correlating with the ability of these antibodies to induce CD31-dependent adhesion (Newton et al., 1997). Additionally, activation of Rap1 by anti-CD31 antibodies was observed in CD31 WT Jurkat transfectants but not CD31 GPI or Y663/686F transfectants (Figure 1C), demonstrating a correlative requirement for the CD31 cytoplasmic tail in the activation of Rap1 and induction of adhesion.

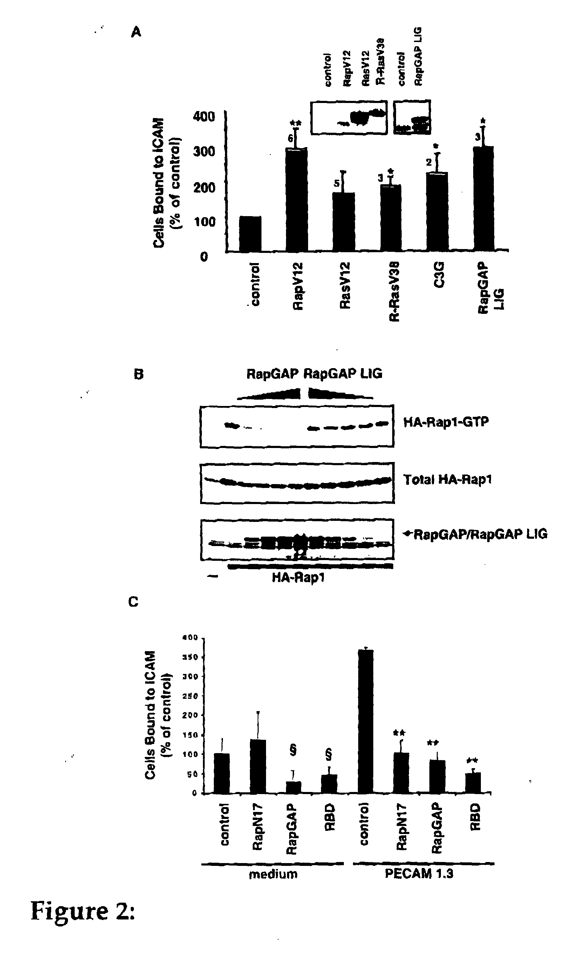

As CD31 stimulation selectively activated Rap1 and Rap1 activation correlated with CD31-dependent adhesion to ICAM and VCAM, we next addressed whether constitutive activation of Rap1 was sufficient to induce adhesion. Jurkat T cells transiently transfected with a luciferase reporter plasmid and indicated cDNA constructs were allowed to bind to immobilized recombinant ICAM and adhering transfected cells detected by measurement of luciferase activity. T cells expressing constitutively active Rap1 (RapV12) or the Rap1 GEF C3G (Tanaka et al., 1994) displayed approximately 3.5- and 3-fold increases in adhesion to ICAM, respectively (Figure 2A). C3G-induced spreading and adhesion on fibronectin has been previously observed in 32D cells, although involvement of Rap1 was not examined and Ras, R-Ras, or other GTPases were implicated (Arai et al., 1999). Expression of either the active form of Ras (RasV12) or R-Ras (R-RasV38) resulted in a lower but detectable induction of adhesion (1.5-2 fold). However, RasV12-stimulated adhesion was not statistically significant, RasV12 stimulated adhesion in only two of five experiments, and RapV12-induced adhesion was always higher than that induced by RasV12 and R-RasV38, even though the latter two constructs were expressed at significantly higher levels than RapV12 (Figure 2A, left inset). Introduction of a RapGAP (Rubinfeld et al., 1991) mutant (RapGAP-LIG), containing a substitution of amino acids 284-286 in the RapGAP arginine finger critical for RapGAP catalytic activity (RKR, mutated to LIG) which unlike wild-type RapGAP displays no GAP activity toward Rap1 (Figure 2B), increased the number of ICAM-bound cells to a similar level as observed with RapV12 and C3G. RapGAP-LIG may perturb Rap1 signaling by displacing endogenous RapGAP from a subcellular localization required for down-regulating Rap1 or may constitutively associate with basally activated Rap1 and serve as an effector protein linking Rap1 to an as yet unidentified target.

We next examined if inhibition of Rap1 signaling would abolish CD31-dependent adhesion to ICAM. We therefore introduced a putative dominant negative mutant of Rap1, RapN17, into cells. RapN17 did not affect basal adhesion of Jurkat to ICAM, but completely abolished CD31-dependent increases in adhesion (Figure 2C). Although the RapN17 mutation was modeled on the corresponding dominant-negative mutation in H-Ras (Medema et al., 1991), RapN17 is not considered to function as a strict dominant-negative protein. Whereas RasN17 blocks Ras signalling by sequestering Ras GEFs, RapN17 fails to bind to Rap1 GEFs in *vitro* (van den Berghe et al., 1997), and fails to inhibit EGF-dependent activation of cotransfected wild-type Rap1 in COS-7 cells (R.M.F.W. and J.L.B., unpublished observation).

Because of uncertainties in the mechanism by which RapN17 might block CD31-stimulated adhesion, we sought independent confirmation that Rap1 was required for CD31-induced adhesion of Jurkat to ICAM. Transient expression of the Rap1-specific GAP RapGAP resulted in a strong reduction of basal adhesion to ICAM, and significantly reduced CD31-dependent adhesion (Figure 2C). This result is compatible with the notion that a Rap1GAP should decrease levels of GTP-bound Rap1 (see Figure 2B) but should still allow ligand-induced activation of Rap1. Finally, we used the Rap1 binding domain of RalGDS, RBD, which binds with high affinity and specificity to GTP-bound Rap1 *in vitro* (Franke et al., 1997). This fragment is hypothesized to block effector binding to Rap1. Expression of RBD significantly abolished both basal and CD31-induced adhesion to ICAM (Figure 2C). Thus, using three independent strategies for interfering with Rap1 signaling, overexpression of inactive Rap1, Rap1-specific RapGAPs, and an isolated RBD of a Rap-binding protein, these experiments demonstrate a critical role for Rap1 in mediating CD31-induced adhesion to ICAM.

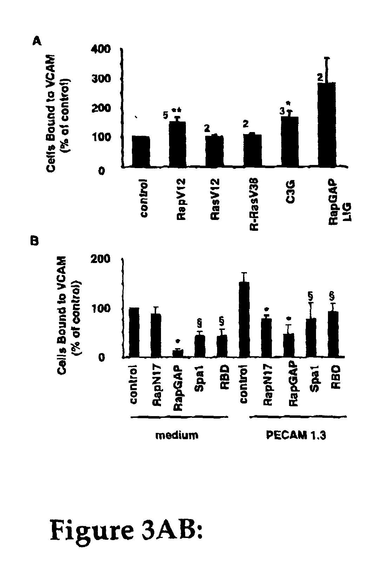

To address whether Rap1 selectively regulated b2 integrins (LFA-1) on Jurkat, or was also coupled to the regulation of b1integrins (VLA-4), we examined the effects of activating and inactivating mutants of Rap signaling on Jurkat adhesion to VCAM. Stimulation of CD31 resulted in a smaller induction of adhesion to VCAM (1.5-2 fold increase) (Figure 3A) than observed to ICAM, in part due to the higher basal level of adhesion observed on VCAM (typically 30-50%, compared to 10-20% on ICAM). A similar increase was also observed in cells expressing RapV12, C3G, and mutant RapGAP-LIG, but not RasV12 or R-RasV38. Conversely, RapN17, RapGAP, and a second GAP for Rap1, Spa1 (Kurachi et al., 1997), significantly reduced basal Jurkat adhesion to VCAM, and strongly blocked CD31-dependent adhesion (Figure 3B).

It has been previously reported that Ras regulates TCR-stimulated lymphocyte adhesion to ICAM (O' Rourke et al., 1998; Tanaka et al., 1999). As we failed to detect Ras activation by CD31, and RasV12 did not induce a clear increase in adhesion to ICAM or VCAM, it is unlikely that Ras mediates the main signalling pathway from CD31 stimulation to integrin-mediated adhesion. However, overexpression of RasN17 did inhibit CD31-dependent adhesion to VCAM (data not shown), suggesting that Ras might also contribute to adhesion responses.

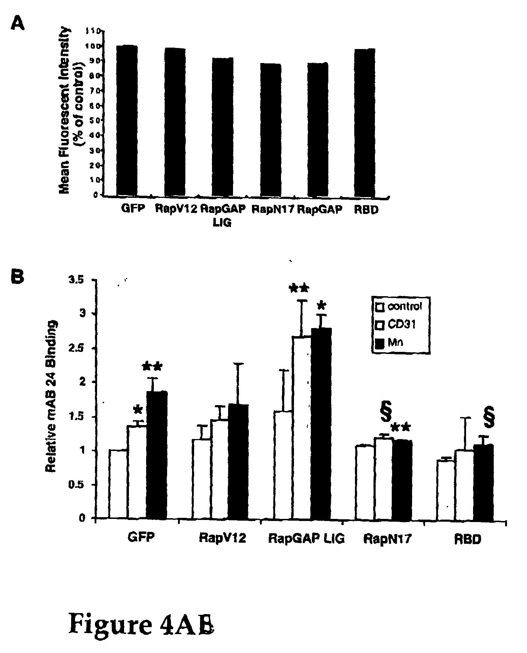

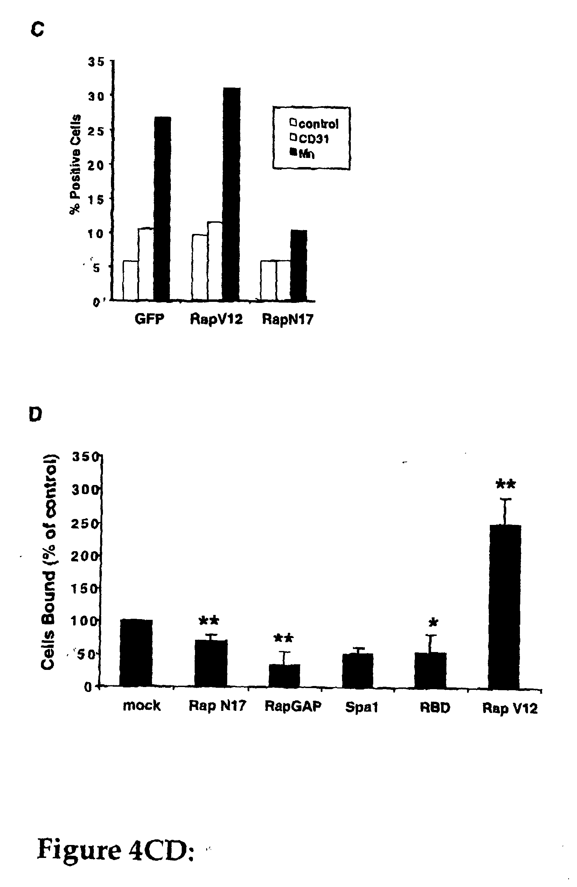

Integrin-dependent adhesion can be regulated by changes in integrin surface expression, integrin surface distribution (avidity), or induction of conformational changes which increase integrin ligand affinity (Stewart and Hogg, 1996). To investigate at which level Rap1 might influence cell adhesion, we first examined if Rap signalling affected LFA-1 expression levels. Jurkat cells were cotransfected with EGFP as a reporter marker, along with indicated Rap1 signalling pathway cDNA constructs. Staining of transfected cells with anti-b2 integrin antibody revealed that overexpression of RapV12, RapN17, RBD, RapGAP or RapGAP LIG failed to influence surface expression levels of LFA-1 (Figure 4A). CD31 expression levels were also unaffected (data not shown). Similarly, RapV12, RapGAP LIG, RapGAP and RapN17 failed to induce LFA-1 surface clustering, as detected with the NKI-L16 antibody (data not shown), although active RasV12 induced clustering of LFA-1, as previously reported (Tanaka et al., 1999).

The anti-aL integrin antibody mAB 24 recognizes a conformation-dependent ligand-induced epitope on LFA-1, and induction of the mAB 24 epitope correlates with increased LFA-1 affinity for ICAM (Dransfield and Hogg, 1989). TCR and TPA-induced mAB 24 epitope expression have previously been found to be dependent on the presence of ICAM, suggesting that signals generated by TCR and TPA facilitate weak basal interactions between LFA-1 and ICAM, stabilizing LFA-1 in a high-affinity conformation (Cabanas and Hobb, 1993). This conformational change can also be induced by LFA-1 exposure to Mn²⁺, independently of "inside-out" signalling. We found that anti-CD31 stimulation of Jurkat, or Jurkat cells transfected with GFP alone, resulted in an approximate 1.5-fold increase in mAB 24 expression (data not shown and Figure 4B), while a 2.25-2.5-fold increase was observed in Mn²⁺-treated cells. Incubation of cells with mAB 24 at 4°C abolished CD31 and Mn²⁺ -induced mAB 24 expression to background levels (data not shown), consistent with the temperature-sensitivity of the mAB 24 epitope (Dransfield and Hogg, 1989). Although RapV12 expression had no statistically significant effect on basal or induced mAB 24 epitope expression, RapN17 and RBD abolished induction of the mAB 24 epitope by CD31 and Mn²⁺. Conversely, RapGAP LIG enhanced CD31- and Mn²⁺- induced expression of the mAB 24 epitope. RapN17 also inhibited the increase in the percentage of mAB 24 -reactive cells observed following CD31 or Mn²⁺ treatment (Figure 4C). Thus, our studies suggest that while Rap does not directly stimulate activation of LFA-1, signalling via Rap1, or basal activation of Rap1, appears to be a requisite factor for stabilization of the active conformation of LFA-1. Moreover, our finding that RapN17 blocks direct conformational changes induced by extracellular Mn²⁺ may suggest that inactive Rap may act to lock LFA-1 in an inactive conformation. Consistent with this model, overexpression of RapN17, RapGAP or RalGDS RBD significantly blocked Mn²⁺-induced adhesion of Jurkat cells to immobilized ICAM, while RapV12 augmented the induced cell adhesion (Figure 4D).

Our report is the first example of requisite involvement of Rap1 in coupling cell surface receptor stimulation to integrin-mediated adhesion. Previously it was shown that Rap1 is activated after stimulation of a large variety of cell surface receptors, including B and T cell antigen receptors, chemokine and cytokine receptors (including fMLP, PAF, and GM-CSF), and receptors for platelet agonists (thrombin, thromboxane A2 and ADP) (reviewed in Bos, 1997). Many of these receptors have been implicated in the control of integrin-mediated adhesion.

### Experimental procedures example 1

Hemagglutinin (HA)-tagged Rap1 (Rap), RapV12, and H-RasV12 in the mammalian expression vector pMT2HA, pRSV, and pRSV-RapN17 have been previously described (Zwartkruis et al., 1998). pMT2HA-R-RasV38 was generated by subcloning a full length *Nco I* (Klenow-filled) /*Xho* I fragment of R-RasV38 into *Not I* (mung bean nuclease-digested) /*Xho* I-digested pMT2HA. A *Sal* I/*Not I* fragment encoding amino acids 200-297 of human RalGDS was amplified by PCR to generate pMT2HA-RalGDS-RBD. pMT2-HA-RapGAP was generated by subcloning full-length RapGAP (provided by Dr. Paul Polakis) as a *Sal I*/ *Bgl II* fragment into pMT2HA. Catalytically inactive RapGAP LIG was generated by mutating RapGAP amino acids 284-286 (RKR) to LIG using the Stratagene Quick-site-directed mutagenesis kit. All PCR-generated sequences were checked by DNA sequencing. pCAGGS-C3G and pSR-His-tagged Spa1 were provided by Drs. Michiyuki Matsuda (NIH, Tokyo) and Masakazu Hattori (Kyoto University), respectively.

Jurkat T cell lines were maintained as previously described (Reedquist and Bos, 1998). The Jurkat line JHM1 2.2 was provided by Dr. Doreen Cantrell, with kind permission of Dr. Art Weiss. Jurkat cells used to generate CD31 WT, CD31 GPI, and Y663/686F stable transfectants were provided by Dr. D. Samson (University of Bath). CD31-positive and -negative variants were established by six rounds of MACs-sorting of parental Jurkat with anti-CD31 antibody 10B9. The negative variant was transfected with CD31 WT, CD31 GPI, or CD31 Y663/686F cDNA in pCDNA3 (Jackson et al., 1997; Newton et al., 1997) and stable polyclonal lines established by selection in G418 (1 mg/ml) and FACS sorting. Jurkat cells were transientally transfected by electroporation with 35 µg plasmid DNA. Jurkat cells (1.2 x 10⁷ cells/ml in 0.4 ml complete media) were pulsed at 250 V and 960 µF with 5µg TK-luciferase plasmid DNA, construct plasmids as indicated in figure legends, and added vector plasmid to keep DNA amounts constant. 24 hours post-transfection, cells were transferred to serum-free media and used 42-48 hours post-transfection for adhesion assays. Subconfluent A14 and COS-7 cells were transfected by calcium phosphate precipitation as previously described (Zwartkruis et al., 1998).

Anti-CD3 antibody T3b was kindly provided by Dr. Hergen Spits (Netherlands Cancer Institute, Amsterdam). Anti-integrin b2 antibody L15, activating anti-integrin µ1 antibody TS2/16 and activating anti-integrin b2 KIM185 have been previously described (Andrew et al., 1993; Van de Wiel-van Kemenade et al., 1992). mAB 24, recognizing a ligand-induced epitope of µL, was kindly provided by Dr. Nancy Hogg (ICRF, London). Anti-CD31 antibodies PECAM 1.2 and PECAM 1.3 were generous gifts from Dr. Peter Newman. Anti-CD31 antibodies 2H8, WM59, GI18 and 9G11 have been previously described (Newton et al., 1997; Yan et al., 1995). Other monoclonal antibodies used in these studies were anti-Rap1, RalA and Ras antibodies (from Transduction Laboratories). Rabbit polyclonal anti- Rap2 and R-Ras antibodies were from Santa Cruz.

Purification of GST-RBD fusion proteins and their use in detecting activated Rap1, Rap2, Ras, and R-Ras by precipitation, SDS-PAGE and immunoblotting have been previously described (de Rooij and Bos, 1997; Franke et al., 1997; Reedquist and Bos, 1998). Detection of bound radiolabelled GDP and GTP to Rap1 was also as previously described (Zwartkruis et al., 1998).

For adhesion assays, transiently transfected Jurkat cells were harvested, washed and resuspended in TSM media (20 mM Tris, pH 8, 150 mM NaCl, 1 mM CaCl₂, 2 mM MgCl₂). 96-well Costar Maxisorp plates were coated overnight at 4°C with goat anti-human IgG antibodies (Jackson, 4 µg/ml in TSM), washed, blocked 30 minutes at 37°C with 1% bovine serum albumin(BSA)/TSM, followed by incubation 1 hour at 37°C with 50 ng/ml recombinant ICAM-1/ or VCAM-1/ human IgG Fc fusion proteins. 50 µl cell suspension was mixed with 50 µl TSM or TSM with stimuli indicated in Figure Legends. Cells were allowed to adhere for 30-60 minutes, and nonadherent cells removed with warm 0.5%BSA/TSM. Adherent cells were lysed and subjected to luciferase assays as previously described (Medema et al., 1992). Expression of transfected constructs was confirmed by immunoblotting of total cell lysates. Cells bound were calculated as in Figure Legends, and were corrected for transfection efficiency and non-specific effects of constructs by measuring luciferase activity of total input cells. Comparison of effects of cDNA construct overexpression on adhesion were made by paired t test or unpaired (Student's) t test as appropriate. Fluorescent labelling of stably transfected cells with 2'7'-bis-(2-carboxyethyl)-5-(and-6)-carboxy fluorescein acetoxymethyl ester (Molecular Probes) and measurement of adherent cells with a Fluoroscan Ascent fluorescent plate reader (Lab Systems) has been previously described (Newton et al., 1997).

Flow cytometric analysis of transfected cells was performed by cotransfecting Jurkat JHMI cells with lmg pCMV-EGFP C1 plasmid (Promega) and the indicated cDNA constructs. Following overnight serum-starvation, cells were equilibrated in 0.5% BSA/1mM CaCl₂ /PBS (FACS Buffer) and left unstimulated, or stimulated for 30 minutes with plate-immobilized anti-CD31 antibody 2H8 (10 mg/ml). Cells were harvested, resuspended in FACS Buffer containing primary antibodies (10 mg/ml) and incubated for 30 minutes on ice, or 37°C for mAB 24 epitope expression. For mAB 24 staining, additional sets of transfected cells were coincubated with 400 mM MnCl₂ during primary antibody staining. Cells were washed with FACS Buffer and stained with secondary rabbit anti-mouse RPE-Cy5-conjugated antibodies (Dako). Fluorescence intensity of EGFP-transfected cells was determined using a FACsCaliber flow cytometer and CellQuest software (both from Becton Dickinson).

### Example 2

Guanine nucleotide exchange factors for Rap are regulated by autoinhibitory domains that respond to intracellualr second messengers.

A number of guanine-nucleotide exchange factors (GEFs) have been identified which mediate the activation of Rap1. The first described Rap1GEF, C3G, is found in a complex with the proto-oncogene product c-Crk and may activate Rap1, as a consequence of complex formation and translocation, induced by receptor tyrosine kinase signaling. CalDAG-GEFI has calcium-binding EF-hands and a domain that resembles C1-type DAG-binding domains and may explain the activation of Rap1 by these two second messengers. Recently, another type of GEF for Rap1, called PDZ-GEF1 or nRapGEP or RaGEF was described. This GEF contains, in addition to the catalytic region, a Ras-binding domain (RED) that may interact directly with Ras and Rap1 in vitro, a PDZ domain that drives membrane association and a domain that is related to cAMP-binding domains (RCBD), but does not bind cAMP. Another RapGEF is Epac (exchange protein directly activated by cAMP), because this GEF represents a novel target for cAMP, independent from the classical target protein Protein Kinase A (PKA) [de Rooij, 1998; Kawasaki, 1998]

Here we have studied the regulation and function of the different Epac family members in more detail. First, we observe that all three members activate in addition to Rap1 also the close relative Rap2. Secondly, we identified an additional putative cAMP-binding site in Epac2, located N-terminal to the DEP domain. Thirdly, mutant analysis revealed that the cAMP-binding domains proximal to the catalytic domains in Epac1 and Epac2 (the B-sites) function as inhibitors of the GEF domains in the absence of cAMP. Fourthly we show that CalDAG-GEF is also regulated by an auto-inhibitory domain which is modelated by calcium binding. Finally we show that PDZ-GEF can be regulated by a small molecule present in Jurkat cell lysate

Epac family members activate both Rap1 and Rap2

Currently, the Epac family of GEFs consists of three members, Epac1 and Epac2 that are regulated by cAMP and Repac (Related to Epac), a member, which lacks any apparent regulatory sequences (Figure 1A). Previously, it was shown that these GEFs activate Rap1 *in vivo* as well as *in vitro,* but not the closely related GTPases Ras, R-ras or Ral. We have extended these experiments and found that all three GEFs can directly activate Rap2 as well (Figure 1B). Equal amounts (approximately 100nM) of GST-fusions of the catalytic domains (Figure 1A) were incubated with fluorescent mantGDP loaded Rap1 or Rap2 (100nM) in the presence of excess unlabelled GDP and exchange of guanine-nucleotides was followed in realtime as a decrease in fluorescence. To compare activity of the different GEFs towards Rap1 and Rap2, single exponential curves were fit from which the exchange reaction rates were calculated. These rates were compared to the intrinsic exchange reaction rates of the GTPases measured in the same experiment. From these calculations a fold induction of guanine-nucleotide exchange on Rap1 and Rap2 was derived, which is depicted in Figure 1C. Epac1 activated Rap2 five times more efficiently than Rap1, whereas Epac2 and Repac activated Rap2 three fold less efficiently (Figure 1B). Activation of Rap2 is not a common feature of all Rap1GEFs, since C3G and CalDAG-GEFI (Figure 1B) did not exhibit catalytic activity towards Rap2 *in vitro.*

To validate the results obtained *in vitro,* we investigated whether the Epac family members also activate Rap1 and Rap2 *in vivo.* Cells were transfected with Epac cDNAs together with either Rap1 or Rap2 and stimulated with forskolin and IBMX to raise the level of cAMP. Activation of Rap1 was measured using the previously described activation-specific probe assay in which Rap1GTP is specifically precipitated with the Rap1 binding domain of Ra1GDS. As shown in Figure 1C, all three GEFs activate Rap1. The activation by Epac1 and Epac2 is enhanced by forskolin treatment.

The activation specific probe assay is less suitable for measuring activation of Rap2, due to the high basal level of Rap2GTP in cells. Therefore, we incubated the cells with 32P-labelled orthophosphate followed by precipitation of Rap2, and separation of bound GDP and GTP. We observed that all three GEFs activate Rap2 *in vivo* (Figure 1D). The activation of Rap2 by Epac1 and Epac2 is enhanced by forskolin treatment. From these results we conclude that all three members of the Epac family, activate both Rap1 and Rap2.

Epac2 has a second, low-affinity binding site for cAMP.

In the completed sequence of the C. elegans genome, only one Epac related gene could be found. This gene encodes a protein that has a putative second cAMP-binding domain at the N-terminus, apart from the reported cAMP-binding domain proximal to the catalytic region. This domain is not present in Epac1, but in Epac2 a similar cAMP-binding site is present (Figure 1A). As judged from primary sequences, this site is similar to the genuine cAMP-binding sites of Epac1, Epac2 and PKA, but distinct from the RCBD domain in PDZ-GEF, a RapGEF which does not respond to cAMP (Figure 2A). We named the N-terminal cAMP-binding domain present in Epac2 and C. elegans Epac the A-site and we named the cAMP-binding domain proximal to the catalytic domains, which is present in Epac1, Epac2 and C.elegans Epac the B-site (Figure 2A). To compare these different sites, we analyzed purified domains (Figure 2A) for in vitro binding to cAMP by isothermal titration calorimetry (ITC). We found that the A-site of Epac2 binds cAMP with an apparent affinity of 87µM, whereas the B-site has an affinity of 1.2 µM, which is comparable to the affinity of 4µM, observed for the cAMP-binding domain of Epac1 (Figure 2B). Apparently, the A-site has a much lower affinity for cAMP as compared to the B-site.

In the regulatory subunits of PKA two cAMP-binding domains are present that cooperatively bind to cAMP, meaning that the binding of cAMP to one site influences the affinity of the second site for cAMP. To investigate whether also site A and B in Epac2 may act cooperatively, we measured cAMP-binding affinity to the complete regulatory region of Epac2. Best-fit analysis revealed two binding sites with Kd's of 0.5 and 76 µM, (Figure 2C), which are in the same range as the affinities of the isolated domains. As a final control a mixture of the separately purified A and B cAMP-binding sites of Epac2, in which no cooperativity can occur, was analyzed in the same assay. This yielded exactly the same result as the titration of the complete regulatory domain of Epac2 (Figure 2C lower panel). The data from these measurements are summarized in Figure 2D. We conclude that no cooperativity occurs in binding of cAMP to the regulatory domain of Epac2 and that also in the full length Epac2 protein, the B-site has a much higher affinity for cAMP that the A-site.

Isolated B-sites inhibit the exchange activity of Epac catalytic domains.

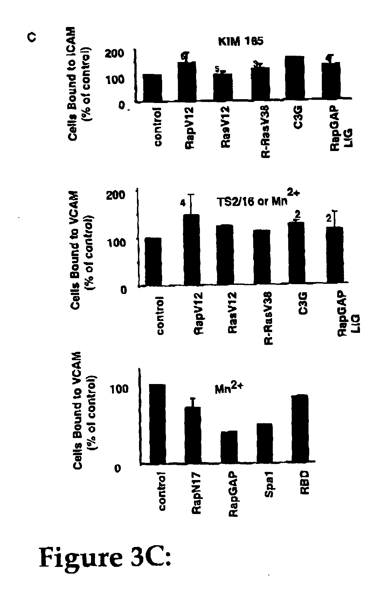

To investigate the role of the different N-terminal domains in the regulation of Epac1 and Epac2 activity by cAMP, we made several deletion constructs (Figure 3A). As shown in Figure 3B, mutant Epac1 (Epac1-DDEP) and Epac2 (Epac2-DDEP) proteins containing, next to the catalytic domain, only the B-site cAMP-binding domain respond to cAMP *in vitro,* like full-length Epac1. However, proteins that lack the B-sites as well as the other N-terminal domains are constitutively active (see Figure 1B). This implies that the B-sites serve as auto-inhibitory domains. Next we investigated whether a direct covalent linkage between the catalytic domain and the B-site is essential for this regulation, or whether they can function as separate domains. We therefore isolated the regulatory domains of both Epac1 and Epac2 and incubated them with the corresponding catalytic domains. As shown in Figure 3C, both regulatory domains completely inhibit the catalytic activity of the corresponding GEF domains, showing that they can form a stable complex that prevents GEF activity. Addition of cAMP abolishes the inhibitory effect. To dissect the role of the two cAMP-binding sites in the regulatory domain of Epac2, purified domains of the A- and the B-site of Epac2 were incubated with the catalytic domain of Epac2. Only the B-site and not the A-site (even at high concentration) inhibits the catalytic domain of Epac2 (Figure 3D). The use of cAMP-binding domain constructs containing also the DEP domain did not alter the ability of the A-site or B-site to inhibit the catalytic activity (data not shown).

The mechanism of Epac regulation is conserved in a subset of RapGEFs.

To investigate whether the cAMP-binding domain of Epac1 can regulate only the catalytic domain of Epac1, we incubated the regulatory domain of Epac1 with the catalytic domains of the other RapGEFs (Figure 4A). As shown in Figure 4B, Epac1-RD inhibited the catalytic activity of both Epac2 and Repac. Interestingly, also the catalytic activity of PDZ-GEF, was inhibited. In contrast, the GEF activity of the catalytic domains of C3G and CalDAG-GEFI was not inhibited. From these results we conclude that the isolated regulatory domain of Epac1, can act as an inhibiting structure for a specific subset of RapGEFs. This indicates that this mechanism of regulation is conserved between Epac and PDZ-GEF. In PDZ-GEF a structure related to cAMP-binding domains (RCBD) is present that probably plays a similar role as the B-sites of Epacs in the regulation of GEF activity. Furthermore, this property is specific for certain cAMP-binding domains only, because neither the A-site of Epac2 (Figure 3C), nor a PKA construct containing both its cAMP-binding domains (Figure 4B) did affect the activity of the catalytic domain of Epac2. Thus we conclude that a specific sequence or structure in the B-sites of Epacs enables these domains to form an inhibitory interaction with the catalytic domains of a subset of RapGEFs.

Interestingly, Epac1-RD was able also to inhibit the catalytic domains of Epac2, Repac and PDZ-GEF. This indicates that the mechanism by which the B-site interacts with the catalytic domain is rather conserved. It is obvious that the presence of both domains in one protein facilitates this mode of regulation, but it could be hypothesized that originally, in early evolution, the two domains were expressed as separate proteins. This question is particularly interesting with respect to Repac, which lacks any intrinsic regulatory domain. It is well possible that a separate regulatory domain, which has not yet been identified, regulates this GEF. Alternatively, Repac is a constitutively active GEF, which is responsible for basal levels of Rap1GTP and Rap2GTP.

We therefore investigated whether CalDAG-GEF was regulated by an auto-inhibitory domain. To that end full length CalDAG-GEF 1 and a CalDAG-Gef mutants lacking the regulatory domain was compared for in vitro activation of Rap1. As shown in Figure the full length construct was significantly less effeicient in activating Rap1. However, when calcium was added to the incubation full length CalDAG-GEF became as efficient as the mutant. From these results we conclude that also for CalDAG-GEF the regulatory domain is an auto-inhibitory domain that is modulated by the binding of a second messenger.

Finally we investigated PDZ-GEF. We had shown previously that deletion of the N-terminal domain related to the cAMP-binding site in Epac (RCBD) results in a more active guanine nucleotide exchange factor for Rap. This suggested to us that perhaps the RCBD is the binding site for a second messenger which regulated PDZ-GEF. Since this domain did not bind to cAMP, we tried lysates from Jurkat cells separated by size exclusion chromatography. Jurkat cells stimulated with serum clearly contain a compound able to activate full length PDZ-GEF. This shows also PDZ-GEF is regulated like Epacs and CalDAG-GEFs, that is by second messengers, which modulate an auto-inhibitory domain.

### Experimental procedures example 2

Constructs used for expression of GEFs and small GTPases in mammalian cells are cloned in the PMT2-SM-HA eukaryotic expression vector. Epac1 constructs are derived from human cDNA and Epac2 constructs are derived from murine cDNA. HA-Epac1-DDEP contains Epac1 lacking amino acids 71-140, which span the DEP domain. For purification of Glutathione S-transferase (GST) -fusion constructs all cDNAs were cloned in pGEX bacterial expression vectors. The catalytic domain of Epac1 contains amino acids 324-881, the regulatory domain of Epac1 (Epac-RD) contains amino acids 2-329, the cAMP-binding domain of Epac1 contains amino acids 149-318 and Epac1-DDEP contains amino acids 149-881. The catalytic domain of Epac2 contains amino acids 460-993, the regulatory domain contains amino acids 1-463, the cAMP-binding site A contains amino acids 1-160, the B-site contains amino acids 280-463 and Epac2-DDEP contains amino acids 280-993. The GST fusion construct of Repac contains amino acids 2-580. The catalytic domain CalDAG-GEFI contains amino acids 3-422. The catalytically active PDZ-GEF1 construct contains amino acids 251-1001. The GST-PKA fusion construct used contained the R1a-subunit of Bovine PKA, lacking amino acids 1-91. Protein production was induced in Bl-21 bacteria using 100 nM IPTG for 20 h at room temperature. After protein production, bacteria were pelleted and lyzed in ice-cold PBS containing 1% Triton-X100 and protease inhibitors. The lysate was sonicated three times for 10 seconds and centrifuged at 10,000 x g to remove insoluble material. GST-fusion proteins we purified from the cleared lysate by batch-wise incubation with glutathione-agarose beads (Sigma), eluted from the beads in buffer containing 50 mM Tris pH 7.5, 100 mM NaCl, 10% glycerol and 10mM glutathione and dialyzed for 20 hours in the same buffer without glutathione. When indicated, proteins were cleaved from the GST-tag by incubation with thrombin and purified by gelfiltration. Small GTPases used for in vitro experiments were described elsewhere.

### In vivo activation of Rap.

Cells were transfected with HA-tagged Rap1A or Rap2A and serum starved for 20 hours prior to the activation experiments. Cells were stimulated with forskolin (20 mM) and isobutylmethylxanthine (IBMX) (1 mM) for 10 min. The GTP-bound form of Rap1 was specifically isolated using GST-RalGDS as an activation specific probe as described. Detection on westernblot was by 12CA5 monoclonal antibodies directed against the HA-tag. In vivo labeling experiment for Rap2 were performed as well. Briefly, serum-starved cells were labeled with 32P-orthophosphate for 5 hours. Rap2 was precipitated using 12CA5 antibodies, nucleotides were eluted and separated on PEI-cellulose F thin layer chromatography (TLC) plates. Labeled nucleotides were visualized using a phospho-imager and GTP/GDP ratios were calculated using the program ImageQuant.

### In vitro activation of small GTPases

Briefly, 100 nM of purified GTPase, loaded with fluorescently labeled 2',3'-bis (O) -N-methylantharanoloyl-guanosinediphosphate(mantGDP), was incubated, in the presence of excess unlabelled GDP, with 50 nM of purified GEF unless indicated differently. Release of mGDP was measured in realtime as a decrease in fluorescence. To calculate reaction rates, single exponential functions were fit using the program Grafit3.0 (Eritacus).

### Figure legends

Figure 1. CD31 selectively activates Rap1 but not other Ras family GTPases. A, Jurkat JHMI 2.2 cells were unstimulated (-) or stimulated with anti-CD31 antibody PECAM 1.3 (10mg/ml) for the indicated times or with TPA (100 ng/ml, 5 minutes), lysed and GTP-bound GTPases precipitated with immobilized GST fusion proteins of RalGDS-RBD (for Rap1 and Rap2) or Raf-RBD (for Ras and R-Ras). Bound GTPases were resolved on SDS-PAGE, transferred to PVDF membrane, and detected by immunoblotting with antibodies against the indicated proteins, followed by enhanced chemiuminescence (ECL). B, Antibodies against CD31 extracellular domains regulating cell adhesion activate Rap1. JHMI cells were activated for 2 minutes with medium (-), anti-CD3 antibody T3b (1:200) or the indicated anti-CD31 antibodies (10 µg/ml), and Rap1 activation detected as in A. Fold activation of Rap1 compared to untreated cells was obtained by quantitating pixels from representative blots using Adobe Photoshop software. C, CD31-dependent activation of Rap1 requires CD31 cytoplasmic tail. CD31 WT, CD31 GPI, and Y663/686F Jurkat cell lines were stimulated for two minutes with medium (-) or 10 µg/ml PECAM 1.3 antibody and activated Rap1 detected as in A. Results shown in each panel are representative of three independent experiments.

Figure 2. Rap1 regulates Jurkat adhesion via LFA-1 (b2) Integrin. A, Activation of Rap1 induces Jurkat adhesion to ICAM. Jurkat JHMI cells were transiently transfected with 5µg PG3- TK-luciferase reporter plasmid and empty vector (control) or pMT2-HA- RapV12, H-RasV12, R-RasV38 (each 10 µg), RapGAP LIG (20 µg) or pCAGGS-C3G (10 µg). Cells were allowed to adhere for 1 hour on immobilized ICAM, washed, lysed, and adherent cells quantitated by luciferase assay. Bars represent the average mean binding and standard error % cells bound from 2-6 independent experiments (indicated above bars) performed in triplicate or quadruplicate, as calculated in Figure 1. Typically, 10-20% of control unstimulated cells adhered to ICAM. ** denotes *p* < 0.01, * *p* < 0.05, and § *p* < 0.1 compared with control cells by paired *t* test. Expression levels of transfected GTPases and RapGAP-LIG from a representative adhesion assay were examined by immunoblotting lysates with anti-HA epitope antibody 12CA5 as in Figure 1A (insets). Blots shown in insets are cut from a single film exposure of one immunoblot. B, RapGAP-LIG mutant fails to stimulate GTP-hydrolyis on Rap1. A14 cells were transfected with vector alone, or HA-Rap1 alone or in combination with increasing amounts (0.3, 1, 2.5, and 5 µg) of HA-tagged RapGAP or RapGAP-LIG, as indicated above top panel. Active HA-Rap1 was precipitated from cell lysates with RalGDS-RBD and detected by 12CA5 immunoblotting as in Figure 2 (top panel). Transfection of HA-Rap1 (middle panel) and RapGAP constructs (lower panel) was monitored by immunoblotting of total cell lysates with anti-HA antibodies. C, Rap1 signaling is required for CD31-dependent adhesion to ICAM. WT CD31 cells transfected with pG3-TK-luciferase reporter plasmid and 30 µg of empty vector (control) or the indicated DNA construct were allowed to adhere to immobilized ICAM as in (a) following 30 minutes stimulation with medium or 10 µg/ml anti-CD31 antibody PECAM 1.3 cross-linked with goat anti-mouse antibodies. Cells bound were quantitated using luciferase assay and %cells bound cis indicated Shown is a representative experiment performed in quadruplicate. *p* values are indicated as in A and represent transfected cell populations compared to control unstimulated cells (medium) or CD31-stimulated control cells.

Figure 3. Rap1 mediates CD31-dependent adhesion via b1 (VLA-4) integrins. A, Activation of Rap1 is sufficient to induce adhesion to VCAM. CD31-positive variant Jurkat cells were transfected with 5 µg pG3-TK-luciferase reporter plasmid and empty vector (control), HA-tagged RapV12, H-RasV12, R-RasV38 (10 µg each), C3G (10 µg each) or RapGAP-LIG (20 mg) and allowed to adhere to immobilized VCAM (50 ng/ml). Adherent cells and %cells bound from five (RapV12), three (C3G) or two (RasV12, R-RasV38, and RapGAP LIG) independent experiments performed in triplicate or quadruplicate were quantitated. 30-50% specific adhesion of control cells to VCAM was observed in the presence of medium alone. Cells transfected with RapV12 (** *p* < 0.01) and C3G (* *p*< 0.05), but not RasV12 or R-RasV38 bound at significantly higher levels than control cells. For RapGAP LIG, *p* was < 0.1003. B, Rap1 signaling is required for CD31-dependent adhesion to VCAM. CD31 WT cells were transfected with 5 mg pG3-TK-luciferase and 30 µg empty vector (control), HA-tagged RapN17, RapGAP, RalGDS-RBD or His-tagged Spa1 were allowed to adhere to VCAM as in A in the absence (control, top panel) or presence of cross-linked anti-CD31 antibody PECAM 1.3 (top panel). Paired t tests of transfected cells revealed that cells transfected with RapGAP (* *p*< 0.05), Spa1 and RED (§ *p*< 0.1) had significantly lower adhesion than control unstimulated cells, and that RapN17, RapGAP (*p*< 0.05), Spa1 and RED (*p*< 0.1) significantly blocked CD31-dependent adhesion.

Figure 4. Rap signalling regulates ligand-induced conformational changes in LFA-1. A. Expression levels of LFA-1 b2 integrin on transfected cells. Jurkat JHMI cells were transiently transfected with 1 µg pCMV- EGFP C1 reporter plasmid along with empty vector (GFP) or the indicated cDNA constructs. Cells were sequentially stained with anti-b2 integrin antibody L15 and rabbit anti-mouse Ig-RPE-Cy5 conjugate, and GFP-expressing cells analysed by flow cytometry. Values are presented mean fluorescent intensity (arbitrary units) normalized to 100% for cells expressing GFP alone. B Rap signalling is required for CD31 and Mn²⁺ - induced conformational changes in LFA-1. JHMI cells were transfected as in A and left unstimulated (control) or stimulated 30 minutes with anti-CD31 2H8 antibody (CD31), followed by staining with the ligand-induced binding anti-LFA-1 antibody mAB 24, as in Materials and Methods. Alternatively, unstimulated cells were coincubated with mAB 24 and 400 µM MnCl₂ (Mn). mAB 24 expression was assessed by flow cytometry, and expression levels normalized to 1 for unstimulated GFP-alone cells in each experiment. Data represent the means and standard errors of representative experiments (GFP, n=4; RapV12 and RapN17, n=3; RapGAP LIG and RBD, n=2). Statistically significant differences between control and stimulated cells, and between stimulated transfected cells are noted (*, *p* < 0.01; **, *p* < 0.05; §, *p* < 0.1). C RapN17 blocks mAB 24 induction. Experiment was performed as in B but data represents the % of mAB 24-expressing cells. D Rap signalling regulates Mn²⁺-induced adhesion to ICAM. Jurkat cells were transfected with luciferase reporter plasmid and indicated constructs as in Figure 3 and allowed to adhere to ICAM in absence or presence of 4 mM MnCl₂, and % specific adhesion calculated as in Figure 2, and normalized to 100 for mock-transfected stimulated cells. Data represent the average mean and error of 2-4 experiments with each construct performed in quadruplicate, and statistically significant differences are indicated (*, *p* < 0.01; **, *p* < 0.05).

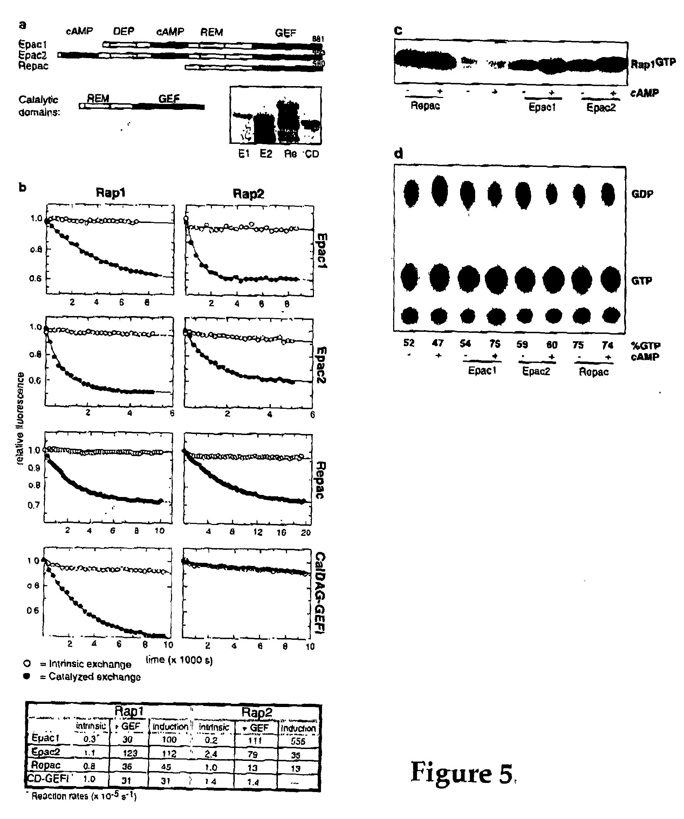

Figure 5. Epac family members activate both Rap1 and Rap2. a; Bacterially expressed GST-fusion proteins containing the catalytic domains of Epac1 (E1), Epac2 (E2), Repac (Re) and CalDAG-GEFI (CD) were purified using glutathione agarose beads, separated by SDS-polyacrylamide gel electrophoresis and stained by coomassie. b; Purified catalytic domains were incubated at approximately 100 nM with purified Rap1A or Rap2A proteins (100 nM) loaded with mantGDP. Decrease in fluorescence was measured at intervals of 15 or 20 seconds. Datapoints shown represent the mean of 20 subsequent measurements. c; Rap1A was cotransfected in Cos-7 cells with the indicated full length GEF constructs. Cells were stimulated with forskolin (20 µM) and IBMX (1 mM) for 10 min and Rap1 activation was measured using GST-RalGDS-RBD as an activation-specific probe. d; HA-Rap2A was cotransfected in Cos-7 cells with the indicated full-length GEF constructs. Cells were labeled with 32P-orthophosphate, Rap2A was immunoprecipitated using 12CA5 monoclonal antibodies, nucleotides were eluted and separated by TLC and GTP/GDP ratios were measured using a phospho-imager.

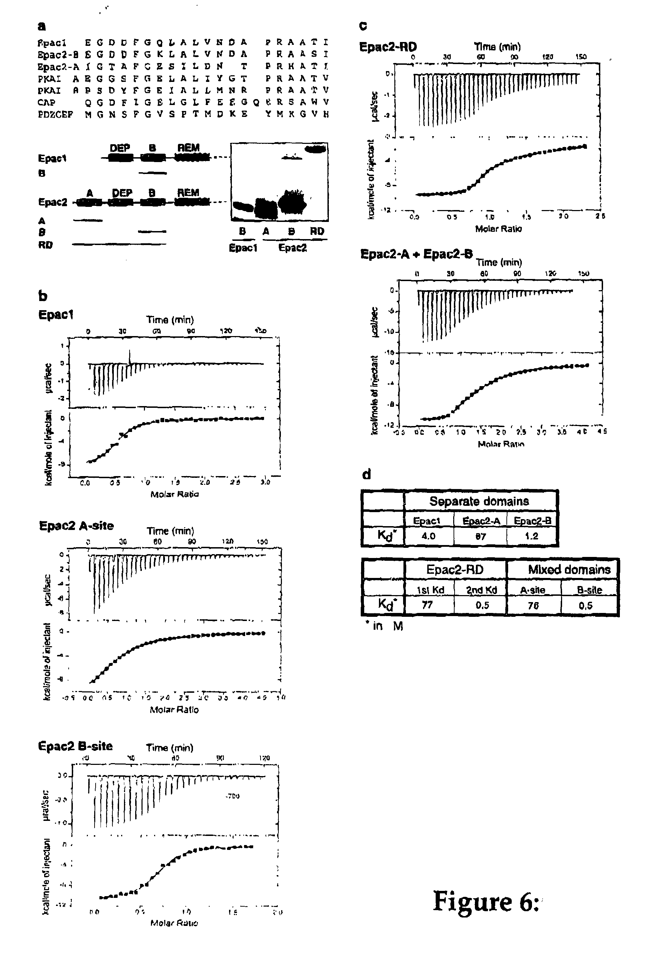

Figure 6. Different cAMP-binding sites in Epac1 and Epac2. a; alignment of different cAMP-binding pockets and schematic presentation and purification of GST-fusion constructs containing the cAMP-binding domains of Epac1 (B) and Epac2 (A and B) and the regulatory domain of Epac2 (RD) (coomassie-stained gel). b,c; The affinities of the isolated cAMP-binding sites in Epac1 and Epac2 were determined by ITC (see experimental procedures). The upper part of the graphs show the time dependent heating power detected after each injection of cAMP. In the lower part, the integrated heating power is normalized to the concentration of injected cAMP and plotted against the molar ratio of the nucleotide and the protein. The conditions used for the different constructs were: 59 µM cAMP-binding domain of Epac1 titrated with 0.72mM cAMP; 200 µM cAMP-binding site A of Epac2 with 3.7 mM cAMP; 34 µM cAMP-binding site B of Epac2 with 0.36 mM cAMP; 68 µM of the complete regulatory domain of Epac2 with 0.68 mM cAMP and in the mixture, 200 µM each of site-A and site-B of Epac2 with 3.7 mM cAMP. d; Kd's (µM) that are calculated from the ITC measurements in b and c are summarized in a table.

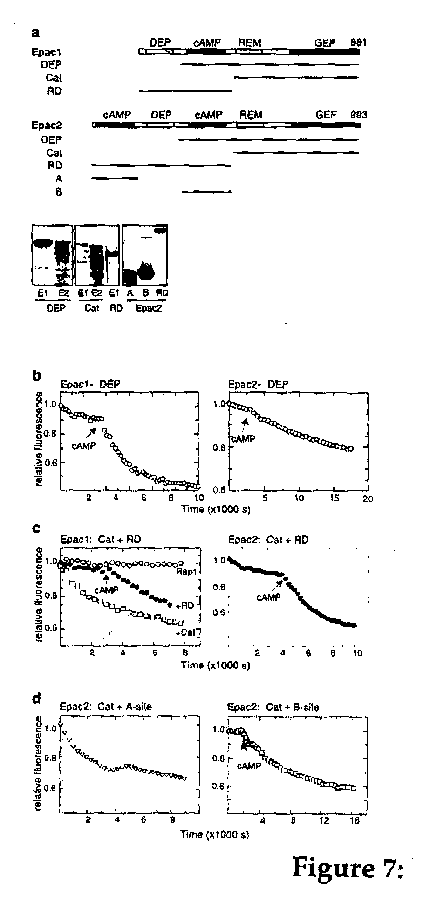

Figure 7. Regulation of Epac catalytic activity by isolated B-sites.
a; Schematic representation and purification (coomassie-stained gel) of the GST-fusion constructs used in b,c and d.
b; Epac1-DDEP or Epac2-DDEP was incubated at approximately 50 nM with purified Rap1A loaded with mantGDP (100 nM). cAMP (10 µM) was added at the indicated timepoints. c; Isolated catalytic domains of Epac1 or Epac2 (50nM) were incubated with their respective regulatory domains (Epac1-RD at 150 nM, Epac2-RD at 3 µM) and mantGDP loaded Rap1A (100nM). cAMP (100 µM) was added at the indicated time points. In the left panel measurements of mantGDP-loaded Rap1 alone (open circles), or in the presence of only the catalytic domain of Epac1 (open squares) are shown for comparison. d; The catalytic domain of Epac2 (50 nM) is incubated with the isolated cAMP-binding domains of Epac 2 (A-site at 200 µM and B-site at 3µM) and mantGDP loaded Rap1A (100 nM). 100 µM cAMP was added to the incubation with the B-site at the indicated timepoint.

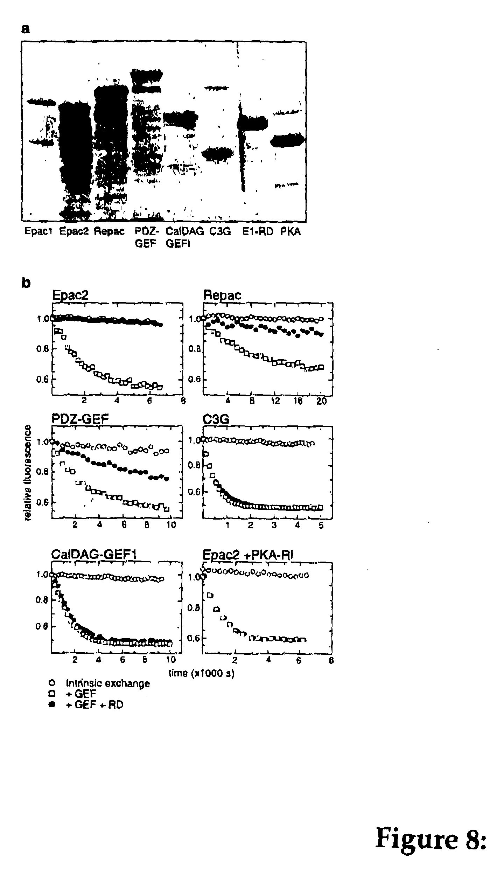

Figure 8. The regulatory domain of Epac1 can block the catalytic domain of closely related RapGEFs.
a; Purification of GST-fusions of the catalytic and regulatory domains used in b. C3G was cleaved from the GST-tag and purified by gelfiltration. b; Intrinsic exchange of mantGDP-loaded Rap1A (100 nM), or Rap2A in the case of PDZ-GEF1, (open circles), activation by the indicated catalytic domains (50 nM) (open squares) and inhibition or lack of inhibition by the presence of Epac1-RD at 150 nM, or 300 nM in the case of C3G and CalDAG-GEFI, or PKA-RIa at 500 nM (black circles).

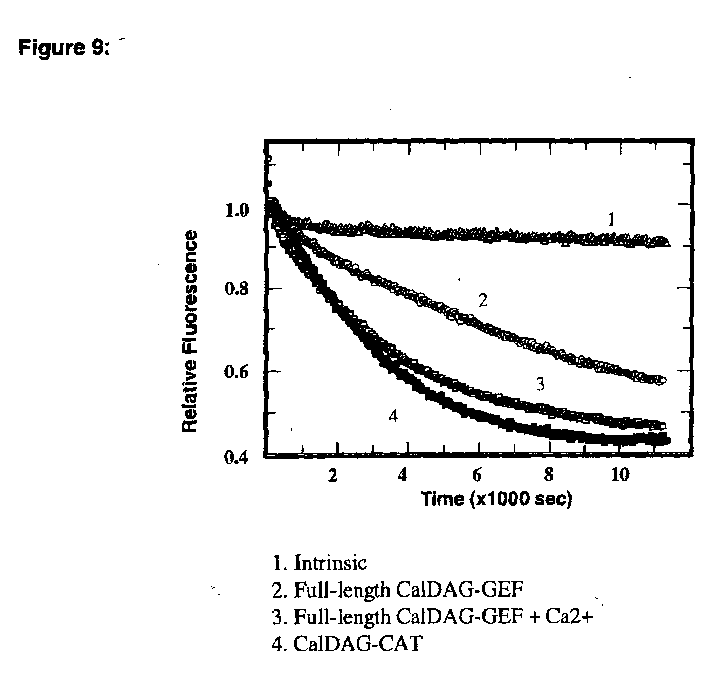

Figure 9. CalDAG-GEF contains an autoinhibitory domain regulated by calcium. Equal amounts of CalDAG-GEF and mutant CalDAG-GEF lacking the DAG and Ca binding domains were incubayed with mantGDP-loaded Rap1 and incubated in the presence or absence of 100 µM calcium.

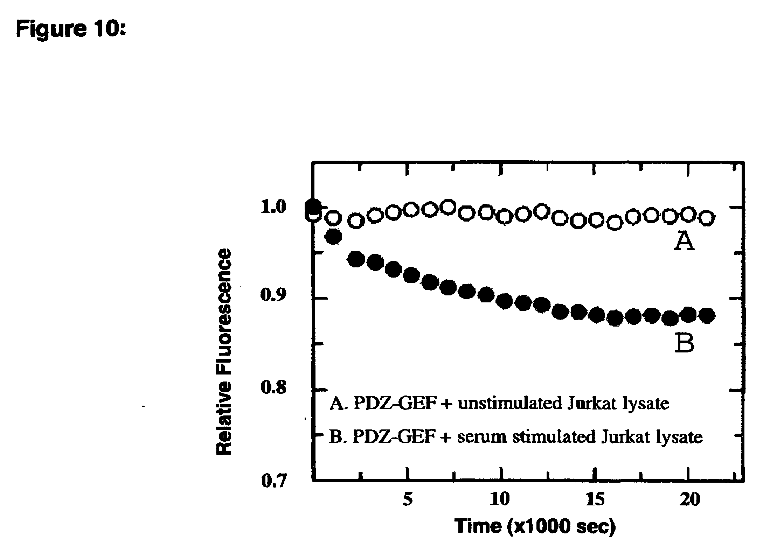

Figure 10. PDZ-GEF contains an auto-inhibitory domain regulated by a factor present in Jurkat lysate and induced by serum.

Figure 11. Currently established guanine nucleotide exchange proteins for Rap

### References

Andrew, D., A. Shock, E. Ball, S. Ortlepp, J. Bell, and M. Robinson. 1993. *Eur. J. Immunol. 9*: 2217-2222.
Bos, J. L. 1997. *Biochim*. *Biophys*. *Acta.* 1333: 19-31.
Bos, J.L. 1998. EMBO J. 17:6776-6782
Cabanas, C., and N. Hogg. 1993. *Proc. Natl. Acad. Sci*., *U.* S. *A.* 90: 5838-5842.
De Rooij, J., and J. L. Bos. 1997. *Oncogene.* 14: 623-625.
De Rooij, J.,N.M. Boenink, M. van Triest5,R.H. Cool,A. Wittinghofer, and J.L. Bos. 1999. *J Boil Chem*.274:38125-38130.
De Vires-Smits, A.M., L. van der Voorn, J. Downward, and J.L. Bos. 1995. *Methods Enzymol.* 255:156-161.
Franke, B.,J.W. Akkerman, and J.L. Bos. 1997. *EMBO J.* 16:252-259
Dransfield, I., and N. Hogg. 1989. *EMBO J.* 8:3759-3765.
Gotoh, T., S. Hattori, S. Nakamura, H. Kitayama, M. Noda, Y. Takai, K. Kaibuchi, H. Matsui, o. Hatase, H. Takahashi, and et al. 1995. *Mol Cell Biol.* 15:6746-6753.
Hughes, P.E. et al., 1997. *Cell.* 88:521-530
Hughes, P.E. and Pfaff, M., 1998. *Trends Cell Biol.* 8:359-364 Kawasaki, H., G.M. Springett, S. Toki, J.J. Canales, P. Harlan, J.P. Blumenstiel, E.J. Chen, I.A. Bany, N. Mochizuki, A. Ashbacher, M. Matsuda, D.E. Housman, and A.M. Graybiel. 1998.*_Proc Natl Acad Sci U S A.* 95:13278-13283.
Kolanus, W., and Seed, B. 1997. *Curr. Opin*. *Cell Biol.* 9:725-731.
Kitayama, H., Y. Sugimoto, T. Matsuzaki, Y. Ikawa, and M. Noda. 1989. *Cell.* 56:77-87.
Kurachi, H., Y. Wada, N. Tsukamoto, M. Maeda, H. Kubota, M. Hattori, K. Iwai, and N. Minato. 1997. *J. Boil. Chem.* 272:28081-28088.
Liao, Y., K. Kariya, C.D. Hu, M. Shibatohge, M. Goshima, T. Okada, Y. Watari, X. Gao, T.G. Jin, Y. Yamawaki-Kataoka, and T. Kataoka. 1999. *J. Boil. Chem.* 274:37815-37820.
Liu, Z.-J., Y. Tanaka, H. Fujimoto, S. Mine, A. Morinobu, H. Yagita, K. Okumura, I. Oishi, J. Udagawa, H. Yamamura, and Y. Minami. 1999. *J*. *Immunol*.163:4901-4908.
Medema, R.H., R. Wubbolts, and J.L. Bos. 1991. *Mol*. *Cell. Boil.* 11:5963-5967.
Newton, J.P., C.D. Buckley, E.Y. Jones, and D.L. Simmons. 1997. *J. Boil. Chem.* 272:20555-20563.
Ohtsuka, T., Y. Hata, N. Ide, T. Yasuda, E. Inoue, T. Inoue, A. Mizoguchi, and Y. Takai. 1999. *Biochem Biophys res Commun.* 265:38-44.
O'Rourke, A.M., H. Shao, and J. Kaye. 1998. *J. Immunol.* 161:5800-5803.
Reedquist, K.A., and J.L. Bos. 1998. *J. Boil. Chem.* 273:4944-4949.
Rubinfield, B., S. Munemitsu, R. Clark, L. Controy, K. Watt, W.J. Crosier, F. McCormick, and P. Polakis. 1991. *Cell.* 65-1033-1042.
Ruoslathi, E. 1999. *Adv. Canc*. *Res.* 65:1-29
Shibayama, H., N. Anzai, S.E. Braun, S. Fukuda, C. Mantal, and H.E. Broxmeyer. 1999. *Blood.* 93:1540-1548.
Stewart, M., and N. Hogg. 1996. Regulation of leukocyte intergrin function: affinity vs advidity. *J. Cell. Biochem.* 61:554-561.
Su, Y., W.R. Dostmann, F.W. Herberg, K. Durick, N.H. Xuong, L. Ten Eyck, S.S. Taylor, and K.I. Varughese. 1995. *Science.* 269:807-813
Tanaka, S., T. Morishita, Y. Hashimoto, S. Hattori, S. Nakamura, T. Takenawa, K. Matuoka, M. Shibuya, T. Kurata, K. Nagashima, and M. Matsuda. 1994. *Proc. Natl. Acad. Sci. USA.* 91:3443-3447.
Tsukamoto, N., M. Hattori, H. Yang, J.L. Bos, and N. Minato. 1999. *J. Boil. Chem.* 274:18463-18469.
Van den Berghe,N., R.H. Cool, G. Horn, and A. Wittinghofer. 1997. Oncogene. 15:845-850.
Van de Wiel-van Kemenade, E., Y van Kooyk, A. J. de Boer, R. J. Huijbens, P. Weder, W. van de Kasteele, C. J. Melief, and C. G. Figdor. 1992. *J. Cell Biol.* 17: 461-470.
Yan, H. C., J. M. Pilewski, Q. Zhang, H. M. DeLisser, L. Romer, and S. M. Albelda. 1995. *Cell. Adhes. Commun.* 3: 45-66.
Zhang, A., K. Vuori, H. G. Wang, J. C. Reed, and E. Ruoslahti. 1996. *Cell.* 85:61-69.
Zwartkruis, F. J. T., R. M. F. Wolthuis, N. M. J. M. Nabben, B. Franke, and J. L. Bos. 1998. *EMBO J.* 17: 5905-5912.

## Claims

1. A method for modulating cell adhesion comprising modulating a protein belonging to the Rap family of small GTPases.

2. A method for modulating cell adhesion comprising modulating a guanine nucleotide exchange factor for a protein belonging to the Rap family of small GTPases.

3. A method according to claim 1 or 2 wherein modulating said cell adhesion comprises modulating integrin-mediated events in said cell adhesion.

4. A method according to claim 3 wherein modulating said cell adhesion comprises modulating the auto-inhibitory domain of a guanine nucleotide exchange factor for a protein belonging to the Rap family of small GTPases.

5. Use of a modulator of a protein belonging to the Rap family of small GTPases for the preparation of a medicament for adhesion or anti-adhesion therapy.

6. Use of a modulator of a guanine nucleotide exchange factor for a protein belonging to the Rap family of small GTPases for the preparation of a medicament for adhesion or anti-adhesion therapy.

7. Use according to claim 5 or 6 wherein adhesion or anti-adhesion therapy comprises modulating integrin-mediated events.

8. Use according to claim 7 wherein said modulator comprises a modulator of the auto-inhibitory domain of a guanine nucleotide exchange factor of a protein belonging to the Rap family of small GTPases.

9. A method for identifying a modulator of a protein belonging to the Rap family of small GTPases or of modulator of a guanine nucleotide exchange factor for a protein belonging to the Rap family of small GTPases comprising
a) providing for binding of a nucleotide or nucleotide analogue to a protein belonging to the Rap family of small GTPases,
b) incubating said protein in the presence of a guanine nucleotide exchange factor of a protein belonging to the Rap family of small GTPases and
c) incubating said protein in the presence of a candidate modulator compound and
d) measuring release of said nucleotide or nucleotide analogue from said protein belonging to the Rap family of small GTPases.

10. A method according to claim 9 wherein activation of said release is measured.

11. A method according to claim 9 wherein inhibition of said release is measured.

12. A modulator obtainable by a method according to anyone of claims 9 to 11.

13. Use of a modulator according to claim 12 in a method for modulating cell adhesion.

14. Use of a modulator according to claim 12 for the preparation of a medicament.

15. Use according to claim 14 for the preparation of a medicament for adhesion or anti-adhesion therapy.

16. A medicament comprising a modulator according to claim 12.
